# EUROPEAN PATENT SPECIFICATION

(11) **EP 3 233 899 B1**
(45) Date of publication and mention of the grant of the patent: **24.06.2020**
(21) Application number: 15830991.4
(22) Date of filing: 14.12.2015
(51) Int. Cl.: C07K 14/655

(54) **A PROCESS FOR THE PREPARATION OF PASIREOTIDE**
VERFAHREN ZUR HERSTELLUNG VON PASIREOTID
PROCÉDÉ DE PRÉPARATION DE PASIRÉOTIDE

(30) Priority: 19.12.2014 IN 6394CH2014
(43) Date of publication of application: 25.10.2017
(73) Proprietor: Auro Peptides LTD, Hyderabad 500084 (IN); Nagana Goud, Agasaladinni, Hyderabad 500084 (IN); Mohammed Abdul, Shafee, Hyderabad, Telangana 500084 (IN); Suresh Kumar, Vadlamani, Hyderabad 500084 (IN); Patil Nilesh, Dagadu, Hyderabad, Telangana 500084 (IN); Mohan Rao, Koppala, Telangana, Hyderabad 500 084 (IN)
(72) Inventor: NAGANA GOUD, Agasaladinni, Telangana Hyderabad 500 084 (IN); MOHAMMED ABDUL, Shafee, Telangana Hyderabad 500 084 (IN); SURESH KUMAR, Vadlamani, Telangana Hyderabad 500 084 (IN); PATIL NILESH, Dagadu, Telangana Hyderabad 500 084 (IN); MOHAN RAO, Koppala, Telangana Hyderabad 500 084 (IN)
(74) Representative: Hamm&Wittkopp Patentanwälte PartmbB
(86) International application number: PCT/IB2015/059576
(87) International publication number: WO 2016/097962

(56) References cited:
- WO-A2-02/10192
- WO-A2-2005/014624
- CN-A- 103 641 894

## Description

### FIELD OF THE INVENTION

The present invention relates to a process for the preparation of Pasireotide of formula **(I)** and its acid addition salts. More particularly the present invention is directed to a process for the synthesis of Pasireotide of formula (I) having purity greater than 99.0% by HPLC using fragment coupling.

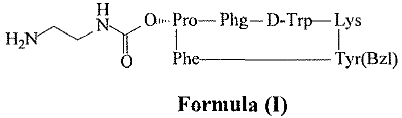

### BACKGROUND OF THE INVENTION

Pasireotide is a somatostatin analog that acts via somatostatin receptors to inhibit the secretion of corticotropin from the pituitary adenoma in patients with Cushing's disease. Pasireotide has a receptor binding profile that is distinct from that of other somatostatin analogues, binding with high affinity to somatostatin receptor subtype 5, which is strongly over expressed in corticotroph adenoma cells. Pasireotide is the first pituitary-directed agent to be approved for use in Cushing's disease.

Pasireotide diaspartate is marketed in USA under the trade name Signifor® for the treatment of adult patients with Cushing's disease for whom pituitary surgery is not an option or has not been curative.

Pasireotide for first time generically disclosed in US 6,225,284 (IN 201579) and specifically disclosed in US 7,473,761 (IN 204073). US '761 patent discloses a method for the preparation of Pasireotide by sequential addition of amino acids on a solid phase to yield linear protected Pasireotide, which is deprotected and cleaved from resin. Thereafter the obtained linear Pasireotide is cyclized using DIPEA & DPPA in DMF, and then the crude peptide was lyophilized and purified using Reverse phase-HPLC to obtain Pasireotide. Pasireotide diaspartate is obtained by treating Pasireotide with aspartic acid in presence of acetonitrile and water.

US 7,615,609 (IN 230296) disclose a process for the preparation of Pasireotide wherein the cyclization occurs between Lysine and Tyrosine in presence of HBTU hexafluorophosphate and HOBT to obtain Pasireotide.

The reported literature for Pasireotide discloses the synthesis by using sequential addition of aminoacids to get linear peptide followed by cyclization. There are certain disadvantages associated with solid phase sequential synthesis such as i) solid phase sequential synthesis does not allow intermediary purification, ii) often fail to produce the desired peptide in sufficient yield and purity.

Considering the importance of Pasireotide as a medicinal product, there is a need to increase the productivity of the said peptide. The present inventors have made Pasireotide by the process, which is simple and industrially scalable with consistent yields. Further, the Pasireotide obtained by the process of the present invention results in higher yield and purity greater than 99.0% by HPLC. Applicant surprisingly found that the Pasireotide can be obtained in high pure (greater than 99.0% by HPLC) form with good yield by fragment based synthesis.

### OBJECTIVE OF INVENTION

An objective of the present invention is to provide a process for preparing Pasireotide, which is simple, robust and industrially applicable.

Another objective of the present invention is to provide a process for preparing Pasireotide, which yields high purity product greater than 99.0% by HPLC.

### SUMMARY OF THE INVENTION

The present invention relates to a process for the preparation of Pasireotide of formula (**I**) and its salts having purity greater than 99.0% by HPLC, 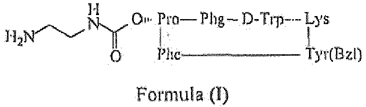 which comprises the following steps:
a) synthesis of at least two protected peptide fragments of Formulae (II) and (III); 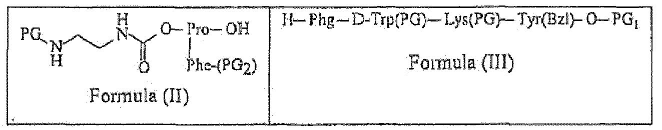
b) coupling of the fragments of step (a) to obtain peptide of formula **(IV);** 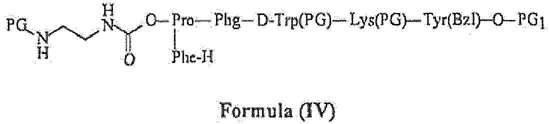
c) converting the peptide of formula (IV) to peptide of formula **(V);** 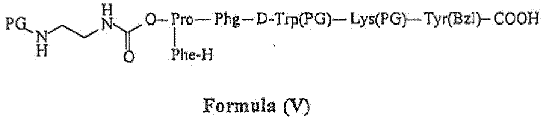
d) cyclizing the peptide of formula (V), followed by deprotection to obtain Pasireotide; and
e) isolation of Pasireotide of formula (I);
   wherein PG represents protecting group and PG₁ represents either acid protecting group or solid-supported resin and PG₂ is a base labile protecting groups.

### BRIEF DESCRIPTION OF ABBREVIATIONS

Boc - t-Butyloxycarbonyl
Bzl - Benzyl
Cbz - Benzyloxy carbonyl
DCC - 1,3-dicyclohexylcarbodiimide
DIC - Diisopropylcarbodiimide
DIPEA - *N,N*-diisopropylethylamine
DMF - *N,N*-dimethylformamide
DMS - Dimethyl sulfide
DMT - dimethoxy trityl
DPPA - Diphenylphosphoryl azide
Fmoc-OSu - N-(9-Fluorenylmethoxycarbonyloxy)succinimide
HBTU - O-Benzotriazole-*N,N,N',N'-*tetramethyluronium hexafluorophosphate
HOBt - 1-Hydroxybenzotriazole
HPLC - High Performance Liquid Chromatography
IPA - Isopropyl alcohol
MMT - Methoxytrityl
Msc - (Methylsulfonyl)ethoxy carbonyl
MTBE - Methyl tert-butyl ether
t-Bu - tert-butyl
TCP - Tetrachlorophthaloyl
TFA - Trifluoro acetic acid
THF - Tetrahydrofuran
TIS - Triisopropyl silane
TLC - Thin Layer Chromatogrpahy
Trt - Trityl
TBTU - 2-(1H-benzotriazol-1-yl)-1,1,3,3-tetramethyluronium tetrafluoroborate
SPPS - Solid phase peptide synthesis
Phe - phenylalanine; Phg - phenylglycine ; D-Trp - D-Tryptophan; Lys - Lysine; Tyr - Tyrosine

### DETAILED DESCRIPTION OF THE INVENTION

In an embodiment, the present invention, Pasireotide and its salts are prepared by fragment based peptide coupling followed by cyclizing the resultant linear peptide rather than sequential addition of amino acids to prepare the linear peptide. Accordingly, the fragment based coupling involve or coupling of fragments having dipeptide (having two amino acid sequence) and tetrapeptide (having four amino acid sequence). These fragments are prepared by either solid phase or solution phase by conventional methods.

In yet another embodiment of the present invention the amino group or the carboxyl group as well as any reactive group in the side chain of the amino acid are suitably protected using conventional protecting groups, Accordingly 4-hydroxy group in proline is suitably substituted and protected.

In still another embodiment of the present invention involves coupling of two suitable peptide fragment of Formula (II) with Formula (III), as indicated in Table-I, to yield corresponding peptide of formula (IV). The fragment coupling can be carried out either by solution phase or solid phase; preferably by solid phase. When the synthesis is effected by solid phase synthesis, the built-up peptide is then removed from the resin in accordance with methods known in the art to yield the peptide of formula (V). Thus the fragment based coupling allows full control over and monitoring the peptide synthesis and yield the required peptide in good yield and purity greater than 99.0% by HPLC.

**Table-1**

| | |
|---|---|
| 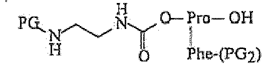 | Formula (II) |
| H-Phg-D-Trp(PG)-Lys(PG)-Tyr(Bzl)-O-PG₁ | Formula (III) |

wherein PG represents suitable acid labile protecting group, PG₁ represents suitable acid protecting group or solid-supported resin may optionally connected through linker and PG₂ base labile protecting groups.

In yet other embodiment of the present invention the group represented by PG₁ in peptide of formula (IV) is removed by conventional methods to yield peptide of formula (V) for example, when PG₁ is solid-supported resin, the peptide of formula (IV) is selectively cleaved from the resin with a mild acidic solutions consisting of different concentrations of TFA, acetic acid-trifluoroethanol-dichloromethane; or hexafluoroisopropanol. The mild acidic solution may be 0.5% TFA in DCM to 20% TFA in DCM, preferably 1% TFA in DCM.

In yet another embodiment of the present invention the peptide of formula (V) thus obtained is cyclized to yield protected Pasireotide. The cyclization of the linear peptide is carried out by using a coupling reagent.

In other embodiment of the present invention the coupling reagent used in the preparation of peptide bond of the present invention is selected from o-(7-azabenzotriazol-1-yl)-1,1,3,3-tetramethyluronium hexafluorophosphate (HATU), o-(benzotriazol-1-0)-1,1,3,3-tetramethyluronium hexafluorophosphate (HBTU), o-(benzotriazol-1-yl)-1,1,3,3-tetramethyluronium tetrafluoroborate (TBTU), benzotriazole-1-yl-oxy-tris(dimethylamino)phosphonium hexafluorophosphate (BOP), benzotriazole-1-yl-oxy-tris-pyrrolidinophosphonium hexafluorophosphate (PyBOP), N,N-bis-(2-oxo-3-oxazolidinyl)phosphonic dichloride (BOP-Cl), bromo-tris-pyrrolidino-phosphonium hexafluorophosphate (PyBroP), iso-butylchloroformate (IBCF), 1,3 dicyclohexylcarbodiimide (DCC), 1,3-diisopropyl-carbodiimide (DIC), 1-(dimethylaminopropyl)-3-ethylcarbodiimide hydrochloride (WSCD1), N-ethoxycarbonyl-2-ethoxy-1,2-dihydroquinoline (EEDQ), isopropylchloroformate (IPCF), 2-(5-norbornen-2,3-dicarboximido)-1,1,3,3-tetramethyluronium tetrafluoroborate (TNTU), propane phosphonic acid anhydride (PPAA), 2-succinimido-1,1,3,3-tetramethyluronium tetrafluoro borate (TSTU), PyClOP, Oxyma pure, TCTU, COMU, HOBt or DEPBT

In still another embodiment of the present invention, the coupling reaction is carried out in presence of a base and in the presence of solvent. The base is organic or inorganic base. The inorganic base comprises potassium carbonate, lithium carbonate, sodium carbonate, sodium ethoxide, sodium bicarbonate, potassium bicarbonate, sodium hydroxide, potassium hydroxide, ammonium hydroxide, and mixtures thereof; the organic base comprises diisopropylamine, N,N-diisopropylethylamine triethylamine, dimethylamine, trimethyl amine, isopropyl ethylamine, pyridine, N-methyl morpholine, piperidine, N,N-dimethylaminopyridine, 1,8-diazabicyclo[5.4.0]undec-7-ene (DBU), and/or mixtures thereof. The solvent comprises dimethylformamide (DMF), dimethylsulfoxide (DMSO), N-Methyl pyrrolidine (NMP), Dimethylacetamide (DMAC), dichloromethane (DCM), methanol, isopropanol, dichloroethane, 1,4-dioxane, tetrahydrofuran (THF), 2-methyl tetrahydrofuran ethyl acetate, acetonitrile, acetone, and the like or mixtures thereof.

In one more embodiment of the present invention, the protected Pasireotide is de-protected by using a mixture of acid (such as TFA), scavenger and solvents. Scavenger is selected from the group comprises Ethanedithiol (EDT), Tri-isopropyl silane (TIS), Triethyl silane (TES), DMS, Anisole, Phenol, Cresol ,thiocresol thioanisole or mixture thereof. Solvent used for deprotection is selected from group comprises water, Dichloromethane (DCM) and Dichloroethane (DCE) or mixture thereof.

In another embodiment of the present invention, suitable protecting groups comprise either amino protecting groups or acid-protecting groups. The amino protecting groups include the following groups but are not limited to these: t-butyloxycarbonyl (Boc), carboxybenzyl (Cbz), o-chlorbenzyloxycarbonyl, bi-phenylisopropyloxycarbonyl, tert.-amyloxycarbonyl (Amoc), α,α-dimethyl-3,5-dimethoxy-benzyloxycarbonyl, o-nitrosulfenyl, 2-cyano-t-butoxy-carbonyl, 9-fluorenylmethoxycarbonyl (Fmoc), 1-(4,4-dimethyl-2,6-dioxocylohex-1-ylidene)ethyl (Dde) and the like. 9-Fluorenylmethoxycarbonyl (Fmoc) and t-butyloxycarbonyl (Boc) is preferably used as the N^{α}-protective group. The amino group protection is carried out by reacting the aminoacid with activated reagent of corresponding protecting group, for example, Fmoc protection is carried out by reacting the amino acid with activated Fmoc-reagents such as the acid chloride derivative (Fmoc-Cl); Fmoc-N-pentafluorophenyl ester (Fmoc-OPfp) and N-(9-fluorenylmethoxycarbonyloxy)succinimide (Fmoc-OSu). Other activating groups are known to those of skill in the art.

In another embodiment of the present invention, the acid protecting group comprises DMT, MMT, Trt, t-Bu and t-butoxy carbonyl.

In yet another embodiment of the present invention the solid phase synthesis of fragment involves the coupling of C-terminal amino acid with a suitable support material (resin). Suitable resins are those which are inert towards the reagents and reaction conditions normally used for the stepwise condensation and cleavage reactions and do not dissolve in the reaction media that are used. Examples of commercially available support materials include 2-chlorotrityl chloride resin, 4-hydroxymethyl-3-methoxyphenoxybutyric acid resin, crosslinked poly N-acryloylpyrrolidone resins, and chloromethylpolystyrene dinvinylbenzene polymer resins. The linkage to the polymeric support can be achieved by reacting the C-terminal Fmoc-protected amino acid with the resin in the presence or absence of coupling reagent. The successive coupling of the protected amino acids can be carried out according to conventional methods in peptide synthesis. The solid phase synthesis is effected in a solvent selected from, but not limited to, ethanol, acetonitrile, N,N-dimethylformamide (DMF), dichloromethane, tetrahydrofuran, N-methylpyrrolidone or similar solvents preferably in DMF at room temperature or elevated temperatures.

In another embodiment of the present invention, the Pasireotide thus obtained is optionally carried out by using conventional purification technique, for example using chromatographic purification techniques such as by using Reverse Phase HPLC.

In yet another embodiment of the present invention, the salts of Pasireotide is selected from one of the acids: aspartic acid, acetic acid, lactic acid, benzoic acid, succinic acid and palmoic acid.

The present invention is illustrated in the following Scheme:

### Synthesis of Fragment I (Solution phase)

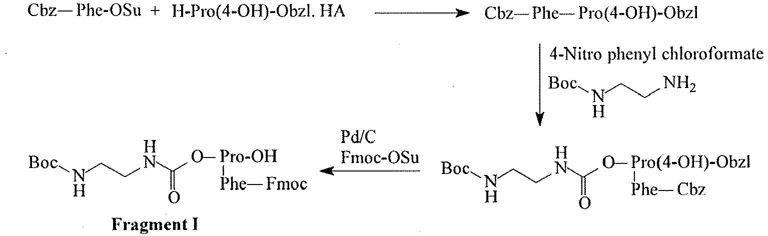

HA is an acid addition salt.

### Synthesis of Fragment II (Solid phase)

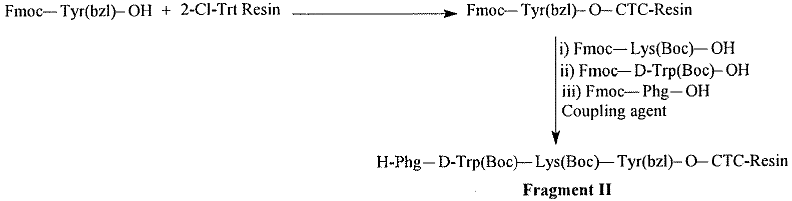

### Coupling of Fragment I and Fragment II

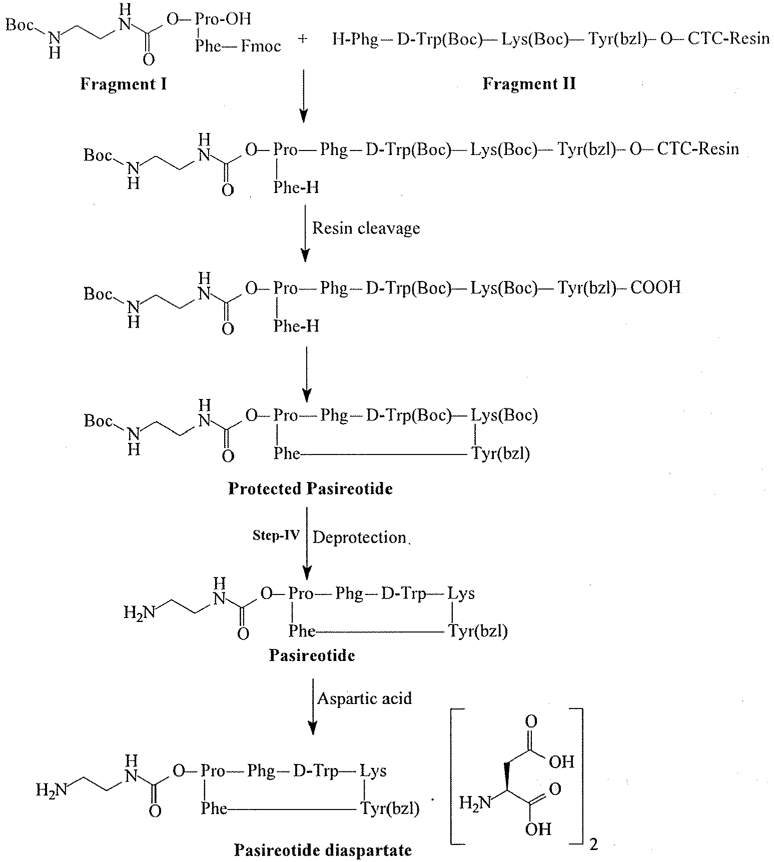

In another embodiment of the present invention, purification of Pasireotide is carried out by successive Reverse Phase HPLC. The RP-HPLC is performed using a commercially available silica gel sorbent as stationary phase. The elution is carried out either by isocratic condition or by gradient mode. Common mobile phases used for elution include, but not limited to, aqueous buffer such ammonium acetate buffer (0.001M to 5M), or water containing acid such as acetic acid (0.001% to 5%), formic acid (0.001% to 5%), TFA (0.001% to 5%), and the like, or any miscible combination of water with various organic solvents like THF, acetonitrile and methanol.

The embodiments of the present invention described above are not intended to be exhaustive or to limit the invention to the precise forms disclosed in the following detailed description. The invention is illustrated with the following examples, which are provided by way of illustration only and should not be construed to limit the scope of the invention in any manner whatsoever.

### EXAMPLE-1

### SYNTHESIS OF Fmoc-Phe-Pro(4-OCONH-(CH₂)₂-NH-Boc)-OH (Fragment I)

### Step-I: Synthesis of Cbz-Phe-Pro(4-OH)-Obzl.

Cbz-Phe-OSu (200.0 g, 1.0eq) was taken in a round bottom flask containing DMF (1.2 L) and cooled the solution to 0-5° C (Solution A). H-Pro(4-OH)-Obzl. HCl (130 g, 1.0 eq) was taken in a separate round bottom flask containing DMF(800 ml) and cooled the solution to 0-5° C. DIPEA (100 ml, 1.1 eq) was added and stirred the reaction mixture for 5 min. This reaction mixture was added to the Solution A and stirred till completion of reaction at room temperature. The progress of coupling was monitored by TLC. After completion of the reaction, brine solution was added to the above reaction mass and extracted twice with ethyl acetate. The ethyl acetate layer was washed with 0.5N HCl, 2% sodium bicarbonate and purified water. The (collected) organic layer was dried with anhydrous sodium sulphate, filtered and the filtrate was evaporated on a rotary evaporator at reduced pressure and temp. 40 ± 5°C, until approximately 80% of ethyl acetate was distilled off. To the reaction mass n-Heptane or di-isopropyl ether was added and stirred to obtain off-white precipitate. The precipitate was filtered and washed with n-Heptane and dried to give Cbz-Phe-Pro(4-OH)-Obzl.
Yield: 176 g

### Step-II: Synthesis of Cbz-Phe-Pro(4-OCONH-(CH₂)₂-NH-Boc)-Obzl

Cbz-Phe-Pro(4-OH)-Obzl(160 g, 1eq) and dimethylaminopyridine (66 g, 1.5 eq) was taken in a round bottom flask containing THF and cooled the solution to 0-5° C, 4-nitro phenylchloroformate (108 g, 1.5 eq) was added slowly and stirred. A solution of Boc-ethylenediamine (280 g, 5 eq) in dichloromethane (1 L) was added to the above reaction mass under nitrogen atmosphere and stirred till completion of reaction. After completion of reaction, 0.5 N HCl was added to the above reaction mass at 0-5° C and extracted twice with dichlormethane.The organic layer was washed with purified water. The (collected) organic layer was dried with anhydrous sodium sulphate, filtered and the filtrate was evaporated completely on a rotary evaporator under reduced pressure at a temperature of 40° C ± 5°C. To residue, di-isopropyl ether was added and stirred to obtain light yellow precipitate. The precipitate was filtered and washed with Di-isopropyl ether and dried to give Cbz-Phe-Pro(4-OCONH-(CH₂)₂-NH-Boc)-Obzl
Yield: 175 g

### Step -III: Synthesis of H-Phe-Pro(4-OCONH-(CH₂)₂-NH-Boc)-OH

A solution of 150 g of Cbz-Phe-Pro(4-OCONH-(CH₂)₂-NH-Boc)-Obzl in methanol (1.5 L) was taken in a autoclave reactor, 30 g of pd/C (10%) was added at room temperature under nitrogen atmosphere. Hydrogen gas was purged and stirred the reaction mixture at room temp. After reaction completion, the catalyst was filtered; filtrate was evaporated to obtain off -white precipitate. The precipitate was washed with MTBE and dried under reduced pressure to give H-Phe-Pro(4-OCONH-(CH₂)₂-NH-Boc)-OH as hygroscopic off-white solid.
Yield: 90 g

### Step -IV: Synthesis of Fmoc-Phe-Pro(4-OCONH-(CH₂)₂-NH-Boc)-OH

75 g of H-Phe-Pro(4-OCONH-(CH₂)₂-NH-Boc)-OH (1 eq) was taken in a flask containing 1,4 dioxane (750 ml) and cooled the solution to 0-5°C, Sodium carbonate(35 g, 2 eq) was dissolved in 750 ml of purified water and added to the above reactor. A solution of Fmoc-OSu (60 g, 1.1 eq) in 1,4 dioxane(250 ml) was prepared and added to the above reaction mass through dropping funnel and stirred for at room temperature. The progress of the reaction was monitored by TLC. After completion of the reaction, acidified the reaction mass with pre-cooled 5% citric acid in water and extracted twice with ethyl acetate.The ethyl acetate layer was washed with purified water.

The (collected) organic layer was dried with anhydrous sodium sulphate, filtered and the filtrate was evaporated completely on a rotary evaporator at reduced pressure and temp. 40° C ± 5° C. Cool this product mass and 1 L of Di-isopropyl ether was added and stirred for 2-3hrs at room temperature to obtain off-white precipitate. The precipitate was filtered and washed with Di-isopropylether and dried to give Fmoc-Phe-Pro(4-OCONH-(CH₂)₂-NH-Boc)-OH(Fragment I)
Yield: 90 g
HPLC purity: ∼97.4 %

### EXAMPLE 2

### SYNTHESIS OF H-Phg-D-Trp(Boc)-Lys (Boc)-Tyr (Bzl)-O-CTC-Resin (Fragment II -Solid phase)

### Step A

2-Cl-Trt Resin (30 gm) was taken in a SPPS reactor, 250 ml of dry dichloromethane was added and allowed it to swell for 10 minutes and drained.

### Step B

A solution of Fmoc-Tyr(Bzl)-OH (29.0 g, 1.5 eq) and DIPEA (27.0 ml, 4 eq) in dry dichloromethane (250 ml) was added to the resin at step A and stirred for two hours at room temperature and drained.

The resin was then capped with methanol (40 %) and DIPEA (10 %) solution in DCM (50 %) for 20 minutes and drained. Thereafter, washed the resin with one bed volume of DMF (2 times), DCM (2 times) and MTBE (2 times) isolated and dried.
Yield: 44.8 g
Loading ∼ 0.9 mmol / g

The above resin was deblocked with 20 % piperidine in DMF(300ml each time) for 10 minutes and 15 minutes and washed with 200 ml of DMF (2 times), IPA (2 times) and DMF (2 times).

### Step C

Fmoc-Lys (Boc)-OH (38.0 g, 2eq.) and HOBT.H₂O (12.2 g, 2eq) were dissolved in DMF (250 ml) and while stirring DIC (16.0 ml, 2.5 eq) was added and stirred the reaction mixture for 5 minutes. It was added to the resin in Step A and stirred for two to three hours at room temperature. The progress of the coupling was monitored by Kaiser Tests. After completion of the reaction, the resin was drained and washed with one bed volume of DMF (2 times).

The above resin was deblocked with 20 % piperidine in DMF(300 ml each time) for 10 minutes and 15 minutes and washed with one bed volume of DMF (2 times), IPA (2 times) and DMF (2 times).

### Step D

Fmoc-D-Trp (Boc)-OH (42.0 g, 2eq.) and HOBT.H₂O (12.2 g, 2eq) were dissolved in DMF (250 ml) and while stirring DIC (16 ml, 2.5 eq) was added and stirred the reaction mixture for 5 minutes. It was added to the resin in Step A and stirred for two to three hours at room temperature. The progress of coupling was monitored by Kaiser Tests. After completion of the reaction the resin was drained and washed with one bed volume of DMF (2 times).

The above resin was deblocked with 20 % piperidine in DMF(300 ml each time) for 10 minutes and 15 minutes and washed with one bed volume of DMF (2 times), IPA (2 times) and DMF (2 times).

### Step E

Fmoc-Phg-OH (30.0 g, 2 eq.) and HOBT.H₂O (12.2 g, 2 eq) were dissolved in THF (250 ml) and while stirring DIC (16 ml, 2.5 eq) was added at low temperature and stirred the reaction mixture for 5 minutes. It was added to the resin in Step A and stirred for two to three hours at room temperature. The progress of coupling was monitored by Kaiser Tests. After completion of the reaction, the resin was drained and washed with one bed volume of DMF (2 times).

The above resin was deblocked with 20 % piperidine in DMF(300 ml each time) for 10 minutes and 15 minutes and washed with one bed volume of DMF (2 times), IPA (2 times) and DMF (2 times) to obtain H-Phg-D-Trp(Boc)-Lys (Boc)-Tyr (Bzl)-O-CTC-Resin (Fragment II).

### EXAMPLE 3

### SYNTHESIS OF H-Phe-Pro(4-OCONH-(CH₂)₂-NH-Boc)-Phg-D-Trp(Boc)-Lys (Boc)-Tyr (Bzl)-O-CTC-Resin.

Fmoc-Phe-Pro(4-OCONH-(CH₂)₂-NH-Boc)-OH (Fragment 1) (55.0 g, 2eq.), HOBT.H₂O (12.2 g, 2eq) and TBTU (26.0 g, 2 eq) were dissolved in DMF (250 ml) and cooled to 0-5° C . While stirring Di-isopropyl ethylamine (28 ml, 4 eq) was added and stirred the reaction mixture for 5 minutes. It was added to the above peptidyl resin in Step A (Fragment II) and stirred for two hours at room temperature. The progress of coupling was monitored by Kaiser Tests. After completion of the reaction, the resin was drained and washed with one bed volume of DMF (2 times).

The above resin was deblocked with 20 % piperidine in DMF(300 ml each time) for 10 minutes and 15 minutes and washed with one bed volume of DMF (2 times), IPA (2 times) and DMF (2 times) to obtain H-Phe-Pro(4-OCONH-(CH₂)₂-NH-Boc)-Phg-D-Trp(Boc)-Lys (Boc)-Tyr (Bzl)-O-CTC-Resin.

Finally the peptide resin was isolated and dried.
Yield: 89.0 g

### EXAMPLE 4

### SYNTHESIS OF H-Phe-Pro(4-OCONH-(CH₂)₂-NH-Boc)-Phg-D-Trp(Boc)-Lys(Boc)-Tyr (Bzl)-OH.

Selective cleavage of 2-chloro trityl resin from the peptide was performed with a mixture 1 % TFA in dichloromethane. The above peptidyl resin was taken in SPPS reactor and treated with a solution of 1 % TFA in DCM (10 ml / g) for 5 minutes at room temp and drained. The filtrate was immediately neutralized with DIPEA (15 % in DCM) under cooling. The above procedure was repeated twice to cleave the peptide from the resin completely. The dichloromethane solution was washed with water (2 times), organic layer was dried with sodium sulphate and concentrated under reduced pressure. Crude protected peptide was isolated by precipitating with MTBE.
Yield: 69.0 g
HPLC purity: 88.3 %

### EXAMPLE 5

### PREPERATION OF PROTECTED PASIREOTIDE (H-Phe-Pro(4-OCONH-(CH₂)₂-NH-Boc)-Phg-D-Trp(Boc)-Lys(Boc)-Tyr(Bzl)-OH)

(46 g, 43 mmol) was taken in a clean and dry 1 L round bottom flask containing DMF (400 ml), TBTU (2.0 eq) and HOBT .H₂O (2.0 eq) was cooled to 0-5° C. DIPEA (3.0 eq) was added and stirred the reaction mixture for 2 hrs.

The progress of the coupling was monitored by TLC. After completion of the reaction water was added to obtain off -white precipitate. The precipitate was filtered and washed with DM water followed by di-isopropyl ether to give protected Pasireotide.
Yield: 43 g
HPLC purity: 77%

### EXAMPLE 6

### PREPERATION OF CRUDE PASIREOTIDE

Deblocking of protected Pasireotide was performed with a mixture 90% TFA + 5% Water + 5% TIS (10 ml / g) for 2 hrs at room temp. The crude peptide (Pasireotide) was isolated by precipitating with MTBE.
Yield: 35 g ; HPLC purity: 74 %

### EXAMPLE 7

### PURIFICATION AND SALT EXCHANGE OF PASIREOTIDE

Crude Pasireotide (35 g) was purified by reverse phase C-18 HPLC using 0.5% aqueous triflouroacetic acid (as buffer A) and 0.5% TFA in 100% acetonitrile (as buffer B). The fractions containing pure peptide were pooled; the organic modifier was removed under reduced pressure. The resulting peptide solution was freeze-dried to isolate white fluffy material as Pasireotide triflouro acetate. The above obtained Pasireotide triflouro acetate was dissolved in purified water and precipitated with 5% sodium carbonate in purified water. The precipitate was filtered and washed with purified water and dried to give Pasireotide (free base). The obtained Pasireotide (free base) was taken into a solution containing two equivalents of aspartic acid in 30% acetonitrile in purified water. The resulting peptide solution was freeze-dried to isolate white fluffy material as Pasireotide di-aspartate.
Yield: 10.2 g ; HPLC purity: > 99.5%
**HPLC Method:** Column: Phenomenex 250×4 mm C-18 ; Flow rate : 1.00 ml/min
Mobile phase: A : TEAP buffer with OPA B: Acetonitrile
**Advantages:** Fragment based synthesis of Pasireotide di-aspartate according to the present invention provides Pasireotide di-aspartate with high yield and purity greater than 99.5% by HPLC over sequential synthesis of Pasireotide di-aspartate. Hence the present invention provides industrially scalable and robust process for the preparation of Pasireotide di-aspartate with purity greater than 99.5%. None of the prior art process provides a process for preparing Pasireotide di-aspartate having purity greater than 99.25% by HPLC. Accordingly the present invention provides a Pasireotide di-aspartate having purity greater than 99.25 % by HPLC.

## Claims

1. A process for the preparation of Pasireotide of formula (I) and its salts having purity greater
than 99.0% by HPLC, 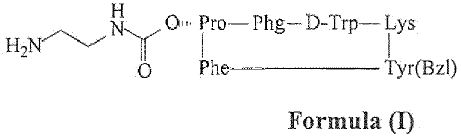 which comprises the steps of:
a) coupling of at least two protected peptide fragments of Formulae (II) and (III) to obtain peptide of formula (IV); 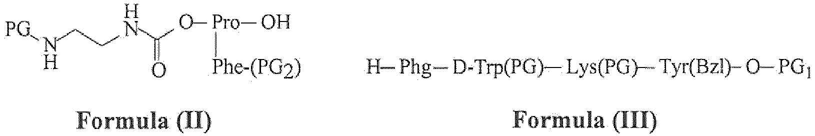 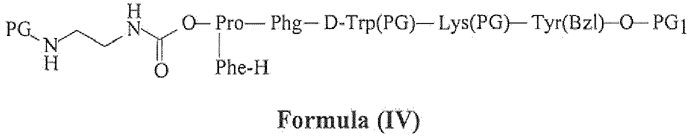
b) converting the peptide of formula (IV) to peptide of formula (V); 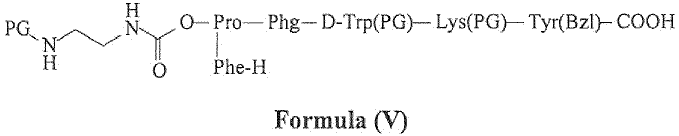
c) cyclizing the peptide of formula (V), followed by deprotection to obtain Pasireotide; and
d) isolation of Pasireotide or its salt;
wherein, PG represents protecting groups and PG₁ represents either acid protecting group or solid-supported resin and PG₂ represents base labile protecting group.

2. The process as claimed in claim 1, wherein the protected peptide fragments are prepared either by solid phase or solution phase synthesis.

3. The process as claimed in claim 1, wherein the coupling of fragments in step (a) and cyclization in step (c) is carried out in presence of a coupling agent.

4. The process as claimed in claim 3, wherein the coupling agent is selected from N,N'-dicyclohexylcarbodiimide (DCC), N,N'-diisopropylcarbodiimide (DIC), o-(benzotriazol-1-yl)-N,N,N'N'-tetramethyluronium hexafluorophosphate (HBTU), 2-(1H-benzotriazol-1-yl)-1,1,3,3-tetramethyluronium tetrafluoroborate (TBTU), benzotriazol-1-yl-tris-pyrrolidino-phosphonium hexafluorophosphate (PyBOP), hydroxysuccinimide (HOSu) and p-nitrophenol (HONp) esters in the presence or in the absence of 1-hydroxybenzotriazole(HOBT) or 1-hydroxy-7-azabenzotriazole.

5. The process as claimed in claim 1, wherein the Pasireotide of formula (I) comprises the steps of :
a) synthesis of protected peptide fragment of formula (II); 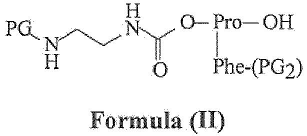
b) synthesis of protected peptide fragment of formula (III)
H-Phg-D-Trp(PG)-Lys(PG)-Tyr(Bzl)-O-PG₁ **Formula (III)**
c) coupling of the fragments of step (a) and step (b) to obtain peptide of formula (IV);
d) converting the peptide of formula (IV) to peptide of formula (V);
e) cyclizing the peptide of formula (V), followed by deprotection to obtain Pasireotide; and
f) isolation of Pasireotide.
wherein PG represents acid labile protecting group, PG₂ represents base labile protecting group and PG₁ represents solid-supported resin.

6. The process as claimed in claim 7, the peptide fragment of Formula (II) is prepared by solution phase synthesis and peptide fragment of formula (III) is prepared by solid-phase synthesis.

7. The process as claimed in claims 1, the suitable protecting groups represented by PG is same or different protecting groups selected from either acid labile protecting group or base labile protecting groups and independently selected from the group comprising of Boc, Cbz, o-chlorobenzyloxycarbonyl, bi-phenylisopropyloxycarbonyl, Amoc, α,α-dimethyl-3,5-dimethoxy-benzylo'xycarbonyl, o-nitrosulfenyl, 2-cyano-t-butoxy-carbonyl, 9- fluorenylmethoxycarbonyl (Fmoc).

8. The process as claimed in claims 1, the suitable acid protecting groups are selected from the group comprising of DMT, MMT, Trt, t-Bu and t-butoxy carbonyl and resin is selected from group comprising CTC, Sasrin, Wang Resin, 4-methytrityl chloride, TentaGel S and TentaGel TGA.

9. The process as claimed in claim 1, wherein the preferable salt of Pasireotide of formula (I) is Pasireotide di-aspartate.

10. The process as claimed in claim 9, wherein the Pasireotide di-aspartate of formula (I) has purity greater than 99.5 % by HPLC.

11. Pasireotide di-aspartate of formula I having purity greater than 99.25 % by HPLC.

## Patentansprüche

1. Ein Verfahren für die Herstellung von Pasireotid der Fromel (I) und seiner Salze mit einer Reinheit von größer als 99,0 % gemessen mit HPLC, 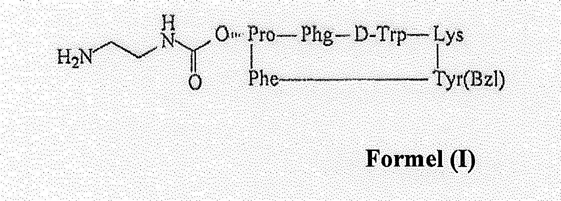 welches die folgenden Schritte umfasst:
a) Kopplung von wenigstens zwei geschützten Peptidfragmenten der Formeln (II) und (III) zur Herstellung eines Peptids der Formel (IV); 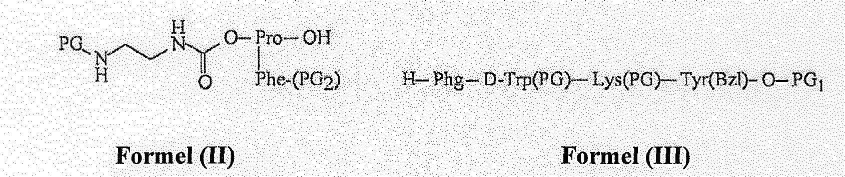 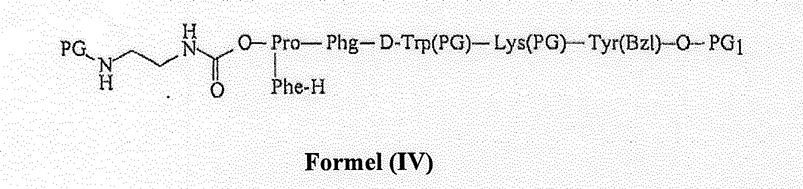
b) Umwandlung der Peptide der Formel (IV) in ein Peptid der Formel (V); 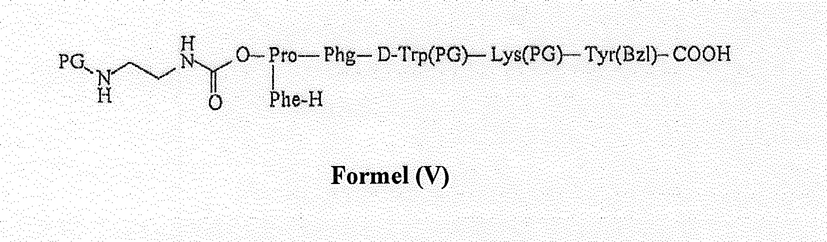
c) Zyklisierung des Peptids der Formel (V), gefolgt von der Entschützung zu Pasireotid; und
d) Isolierung von Pasireotid oder seines Salzes;
wobei PG Schutzgruppen darstellen und PG₁ entweder eine Säureschutzgruppe oder ein Feststoff-unterstützter Harz ist und PG₂ eine basenlabile Schutzgruppe darstellt.

2. Ein Verfahren gemäß Anspruch 1, wobei die gestützten Peptidfragmente entweder durch Festphasen- oder Flüssigkeitsphasensynthese hergestellt werden.

3. Ein Verfahren gemäß Anspruch 1, wobei die Kopplung der Fragmente in Schritt (a) und die Zyklisierung in Schritt (c) in Anwesenheit eines Kopplungsagens ausgeführt wird.

4. Ein Verfahren gemäß Anspruch 3, wobei das Kopplungsagens ausgewählt ist aus N,N'-Dicyclohexylcarbodiimid (DCC), N,N'-Diisopropylcarbodiimid (DIC), o-(Benzotriazol-1-yl)-N,N,N',N'-tetramethyluroniumhexafluorophosphat (HBTU), 2-(1H-Benzotriazol-1-yl)-1,1,3,3-tetramethyluroniumtetrafluoroborat (TBTU), Benzotriazol-1-yl-tris-pyrrolidino-phosphoniumhexafluorophosphat (PyBOP), Hydroxysuccinimid (HOSu) und p-Nitrophenol (HONp) Ester in Anwesenheit oder in Abwesenheit von 1-Hydroxybenzotriazol (HOBT) oder 1-Hydroxy-7-azabenzotriazol.

5. Ein Verfahren gemäß Anspruch 1, wobei das Pasireotid der Formel (I) die Schritte umfasst:
a) Synthese des geschützten Peptidfragments der Formel (II); 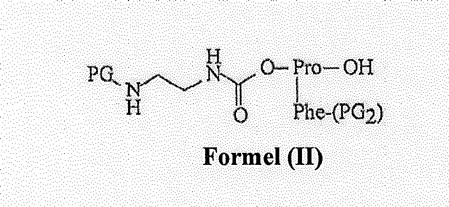
b) Synthese des geschützten Peptidfragments der Formel (III);
H-Phg-D-Trp(PG)-Lys(PG)-Tyr(Bzl)-O-PG₁ **Formel (III)**
c) Kopplung der Fragmente aus Schritt (a) und Schritt (b), um das Peptid der Formel (IV) zu erhalten;
d) Umwandlung des Peptids der Formel (IV) in ein Peptid der Formel (V)
e) Zyklisierung des Peptids der Formel (V), gefolgt von der Entschützung, um Pasireotid zu erhalten und
f) Isolierung von Pasireotid,
wobei PG eine säurelabile Schutzgruppe darstellt, PG2 eine basenlabile Schutzgruppe und PG1 ein Feststoff-unterstützter Hart ist.

6. Ein Verfahren gemäß Anspruch 7, wobei das Peptidfragment der Formel (II) durch eine Flüssigphasensynthese und das Peptidfragment der Formel (III) durch eine Festphasensynthese hergestellt werden.

7. Ein Verfahren gemäß Anspruch 1, wobei die geeignete Schutzgruppe PG ist die gleiche oder eine andere Schutzgruppe, ausgewählt aus entweder säurelabilen Schutzgruppen oder basenlabilen Schutzgruppen und unabhängig voneinander ausgewählt ist aus der Gruppe, umfassend Boc, Cbz, o-Chlorobenzyloxycarbonyl, Biphenylisopropyloxycarbonyl, Amoc, *α,α*-Dimethyl-3,5-dimethoxy-benzyloxycarbonyl, 1-Nitrosulfenyl, 2-Cyano-t-butoxy-carbonyl, 9-Fluorenylmethoxycarbonyl (Fmoc).

8. Ein Verfahren gemäß Anspruch 1, wobei die geeigneten Säureschutzgruppen ausgewählt werden aus der Gruppe, umfassend DMT, MMT, TRT, t-Bu und t-Butoxycarbonyl und der Harz ausgewählt ist aus der Gruppe, umfassend CTC, Sasrin, Wangharz, 4-Methytritylchlorid, TentaGel S und TentaGel TGA.

9. Ein Verfahren gemäß Anspruch 1, wobei das bevorzugte Salz von Pasireotid der Formel (I) Pasireotiddiaspartat ist.

10. Ein Verfahren gemäß Anspruch 9, wobei das Pasireotiddiaspartat der Formel (I) eine Reinheit von größer als 99.5 % (HPLC) aufweist.

11. Pasireotiddiaspartat der Formel (I), das eine Reinheit von größer als 99,25 % (HPLC) aufweist.

## Revendications

1. Méthode de préparation de pasiréotide de formule (I) et de ses sels ayant une pureté supérieure à 99,0 % par HPLC, 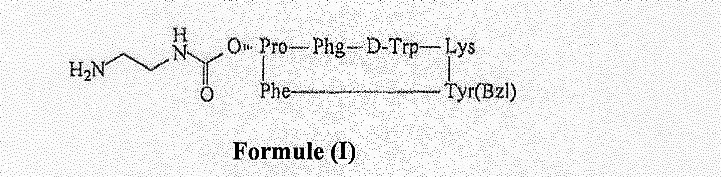 qui comprend les étapes consistant à :
a) coupler au moins deux fragments peptidiques protégés de formules (H) et (III) pour obtenir le peptide de formule (IV) ; 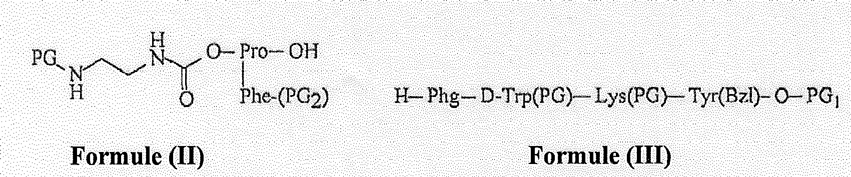 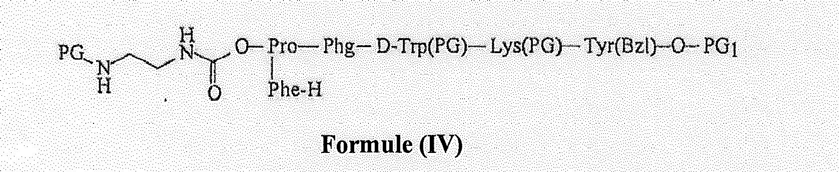
b) convertir le peptide de formule (IV) en peptide de formule (V) ; 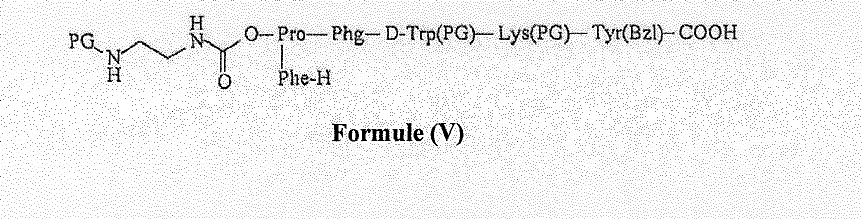
c) cycliser le peptide de formule (V), étape suivie d'une déprotection pour obtenir le pasiréotide ; et
d) isoler le pasiréotide ou son sel ;
dans laquelle PG représente des groupes protecteurs et PG₁ représente soit un groupe protecteur acide soit une résine supportée sur un solide et PG₂ représente un groupe protecteur labile de base.

2. Méthode selon la revendication 1, dans laquelle les fragments peptidiques protégés sont préparés par synthèse en phase solide ou en phase solution.

3. Méthode selon la revendication 1, dans laquelle le couplage de fragments à l'étape (a) et la cyclisation à l'étape (c) sont effectués en présence d'un agent de couplage.

4. Méthode selon la revendication 3, dans laquelle l'agent de couplage est choisi parmi N,N'-dicyclohexylcarbodiimide (DCC), N,N'-diisopropylcarbodiimide (DIC), o-(benzotriazol-l-yl)-N,N,N'N'-hexafluorophosphate de tétraméthyluronium (HBTU), 2-(1H-benzotriazol-1-yl)-1,1,3,3-tétrafluoroborate de tétraméthyluronium (TBTU), benzotriazol-1-yl-tris-pyrrolidino-hexafluorophosphate de phosphonium (PyBOP), hydroxysuccinimide (HOSu) et esters de p-nitro-phénol (HONp) en présence ou en l'absence de 1-hydroxybenzotriazole (HOBT) ou de 1-hydroxy-7-azabenzotriazole.

5. Méthode selon la revendication 1, dans laquelle le pasiréotide de formule (I) comprend les étapes de :
a) synthèse d'un fragment peptidique protégé de formule (II) ; 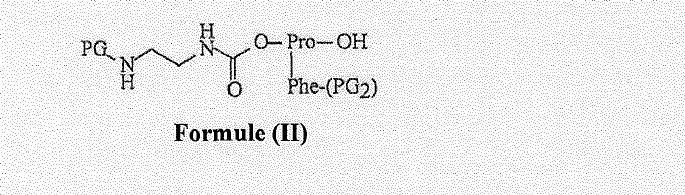
b) synthèse d'un fragment peptidique protégé de formule (III)
H-Phg-D-Trp(PG)-Lys(PG)-Tyr(Bzl)-O-PG₁ **Formula (III)**
c) couplage des fragments de l'étape (a) et de l'étape (b) pour obtenir un peptide de formule (IV) ;
d) conversion du peptide de formule (IV) en peptide de formule (V) ;
e) cyclisation du peptide de formule (V), suivie d'une déprotection pour obtenir le pasiréotide ; et
f) isolation du pasiréotide.
dans PG représente un groupe protecteur labile acide, PG₂ représente un groupe protecteur labile de base et PG₁ représente une résine sur support solide.

6. Méthode selon la revendication 7, le fragment peptidique de formule (II) étant préparé par synthèse en phase solution et le fragment peptidique de formule (III) étant préparé par synthèse en phase solide.

7. Méthode selon la revendication 1, les groupes protecteurs appropriés représentés par PG sont des groupes protecteurs identiques ou différents choisis parmi un groupe protecteur labile acide ou des groupes protecteurs labiles de base et choisis indépendamment dans le groupe comprenant Boc, Cbz, o-chlorobenzyloxycarbonyle, bi-phénylisopropyloxycarbonyle, Amoc, α,α-diméthyl-3,5-diméthoxy-benzylo'xycarbonyle, o-nitrosulfényle, 2-cyano-t-butoxy-carbonyle, 9-fluorényl-méthoxycarbonyle (Fmoc).

8. Méthode selon la revendication 1, les groupes protecteurs acides appropriés sont choisis dans le groupe comprenant DMT, MMT, Trt, t-Bu et t-butoxycarbonyle, et la résine est choisie dans le groupe comprenant CTC, Sasrin, résine Wang, 4-chlorure de méthytrityle, TentaGel S et TentaGel TGA.

9. Méthode selon la revendication 1, dans laquelle le sel préférable du pasiréotide de formule (I) est le di-aspartate de pasiréotide.

10. Méthode selon la revendication 9, dans laquelle le di-aspartate de pasiréotide de formule (I) a une pureté supérieure à 99,5 % par HPLC.

11. Di-aspartate de pasiréotide de formule I ayant une pureté supérieure à 99,25 % par HPLC.


### Sequence listing

<110> AURO PEPTIDES LTD
<120> A PROCESS FOR THE PREPARATION OF PASIREOTIDE
<130> 6394/CHE/2014
<140>
   <141>
<150> IN 6394/CHE/2014
   <151> 2014-12-19
<160> 3
<210> SEQ ID NO 1
   <211> 6
   <212> PRT
   <213> Artificial sequence
<220>
   <223> synthetically generated peptide and appropriate contains protecting groups and substitution as indicated in the specification
<400> 1 
Phe Pro Gly D-Trp Lys Tyr
1                    5
<210> SEQ ID NO 2
   <211> 6
   <212> PRT
   <213> Artificial sequence
<220>
   <223> Pasireotide sequence and contains appropriate substitution
<400> 2 
Phe Pro Gly D-Trp Lys Tyr
1 5
<210> SEQ ID NO 3
   <211> 4
   <212> PRT
   <213> Artificial sequence
<220>
   <223> synthetically generated peptide and appropriate contains protecting groups and substitution as indicated in the specification
<400> 3

Gly D-Trp Lys Tyr
1

### Sequence listing

<110> AURO PEPTIDES LTD
<120> A PROCESS FOR THE PREPARATION OF PASIREOTIDE
<130> 6394/CHE/2014
<140>
   <141>
<150> IN 6394/CHE/2014
   <151> 2014-12-19
<160> 3
<210> SEQ ID NO 1
   <211> 6
   <212> PRT
   <213> Artificial sequence
<220>
   <223> synthetically generated peptide and appropriate contains protecting
   groups and substitution as indicated in the specification
<400> 1 
Phe Pro Gly D-Trp Lys Tyr
1               5
<210> SEQ ID NO 2
   <211> 6
   <212> PRT
   <213> Artificial sequence
<220>
   <223> Pasireotide sequence and contains appropriate substitution
<400> 2 
Phe   Pro   Gly   D-Trp   Lys   Tyr
1                         5
<210> SEQ ID NO 3
   <211> 4
   <212> PRT
   <213> Artificial sequence
<220>
   <223> synthetically generated peptide and appropriate contains
   protecting groups and substitution as indicated in the specification
<400> 3

Gly  D-Trp  Lys  Tyr
 1